# EUROPEAN PATENT SPECIFICATION

(11) **EP 0 591 144 B1**
(45) Date of publication and mention of the grant of the patent: **25.08.1999**
(21) Application number: 90915551.7
(22) Date of filing: 12.09.1990
(51) Int. Cl.: A61F 5/44, A61F 5/441, A61F 5/443, B65D 30/24, B65D 33/16, A61G 9/00, A61B 10/00

(54) **FLUID CONTAINMENT BAG**
FLÜSSIGKEITSSACK
SAC POUR LIQUIDES

(43) Date of publication of application: 13.04.1994
(73) Proprietor: AMERICAN INNOTEK, INC., San Marcos, CA 92069 (US)
(72) Inventor: YOUNG, Ruth, E., Escondido, CA 92025 (US); YOUNG, Daniel, L., Escondido, CA 92025 (US); WARRICK, Richard, E., Encinitas, CA 92024 (US); CASSIDY, Clarence, A., Carlsbad, CA 92009 (US)
(74) Representative: Wilson, Nicholas Martin
(86) International application number: US9005178
(87) International publication number: WO9203994

(56) References cited:
- EP-A- 0 263 315
- DE-A- 1 815 038
- GB-A- 2 016 929
- GB-A- 2 227 728
- US-A- 3 297 152
- US-A- 3 403 715
- US-A- 4 179 367
- US-A- 4 387 713
- US-A- 4 541 117
- US-A- 4 581 763

## Description

### FIELD OF THE INVENTION

The present invention relates to fluid containment bags and particularly to a disposable bag for collection of human bodily fluids such as urine, blood and vomit.

### BACKGROUND OF THE INVENTION

This invention addresses the problem of providing for the collection and disposal of bodily fluid, especially waste liquids, other than with conventional bathroom, collection or sanitary facilities.

There are many situations in which a person finds himself or herself with a need to urinate but without any conventional bathroom facilities readily available. Telephone and electrical cable splicers and installers may work in underground manholes and tunnels and have no access to bathroom facilities without leaving the job site and traveling to a public restroom. Similarly, workers in the field such as telephone and electrical workers are often working at a location remote from bathroom facilities and also must stop work and travel to distant remote facilities several times a day to relieve themselves. In the past it has often been the practice that a person working in a remote or confined location would have no choice but to find a reasonably private location at the job site and urinate there. Such is a highly unsatisfactory situation, however, since it is in violation of many health and environmental regulations, particularly in more recent years when such regulations have become more prevalent.

Similarly, pilots and passengers in many types of private and military planes do not have regular bathroom facilities available to them during long flights. The problem is particularly acute in the case of military pilots who must fly long missions in extremely cramped surroundings and yet must be sufficiently alert to be able to respond instantly to hostile action. The military have, of course, been aware of the problem and the Navy and Air Force have provided pilots with a type of urine collection bag containing a sponge-like material intended to entrap the liquid. It has been found, however, that leakage of significant amounts of urine from such bags is very common, particularly when the pilots perform aerial maneuvers.

The need for a convenient manner of safely and securely collecting and retaining waste liquids for disposal is also apparent in other situations. Those who suffer from motion sickness in vehicles such as cars and airplanes need a satisfactory container to collect and dispose of vomit which results from their motion sickness.

In a different setting, medical personnel must often dispose of significant quantities of patients' blood in surgery, trauma centers or emergency rooms where patients may be bleeding profusely. The current practice of using open containers to collect the blood has proven quite unsatisfactory.

Further, there are circumstances which do not actually involve bodily "waste" but where bodily fluid collection is performed, such as where one may wish to collect a bodily fluid for analysis or examination, as with collection of a urine or blood specimen.

The UK Patent Application No. 2 227 728 discloses a disposable bag for vomit or urine with hand protection consisting of a flattened conical paperboard funnel to which is sealably attached a bag. The funnel has a duck-bill valve attached to its lower end, and the valve is disposed inside the bag. A shroud or cover is sealingly attached to the outer surface of the cover providing protection for the hand of the user of the bag. However, to prevent any content of the bag to escape there is only provided the duck-bill valve which may not work satisfactory especially if the filled bag is inverted, jarred or disposed somewhere not in its upright position.

The US Patent No 4,179,367 relates to a method of thickening urinary tract and intestinal excrement by means of a cross-linked water-swelling polymer. The idea is to mix the bodily fluids or excrement collected in a container with the polymer to thicken the fluids/excrement and to eliminate odors so as to provide easier handling and disposal of the excrement. The specification is silent on which specific containers may be suitable to receive the thickened fluids/excrement and how these container should look like as to safely keep the thickened fluids/excrement.

DE-A-1815038 discloses a containment bag for human bodily fluids having the features set out in the preamble of claim 1 of the present application. In particular that document discloses a disposable urine receptacle for male bed patients in which urine is absorbed in a cellulose material.

There is therefore clearly a need for a convenient and practical fluid collection and containment bag which may be easily used even in a confined environment, which provides secure and complete absorption and retention of the fluids even under external forces which would otherwise tend to disperse the liquid and which can be conveniently and safely disposed of.

### SUMMARY OF THE INVENTION

According to the invention there is provided a containment bag for human bodily fluids, said containment bag comprising:
a bag having a hollow interior defined by two sides meeting at opposite edges, a bottom and a top with said edges and bottom sealed and said top being at least partially open, and
funnel means having an open top, being secured to the bag at the top of the bag and extending downwardly within the interior of the bag to a narrower open bottom whereby fluid entering the open top of the funnel means may be conducted into bag whilst the escape of fluid from within the bag through the funnel means is resisted,
the funnel means being arranged completely inside the interior of the bag;
the funnel means comprising flexible material which is attached at its top to the top of the bag; characterized in that:
gellable hydrophillic polymer material is contained within the bag, said material becoming fully gelled within thirty seconds of contact when the fluid is deposited in the bag, said gelation serving to essentially completely sequester the fluid and to prevent the fluid from thereafter being expelled from the bag;
and closure means for closing the top of said bag after introduction of said bodily fluids into said bag.

The rapid and complete sequestration of the bodily fluids permits the bag to be easily and conveniently used in a variety of circumstances without any possibility that the bodily fluids will become spilled or otherwise expelled from the bag even under severe external forces such as aircraft maneuvers.

### BRIEF DESCRIPTION OF THE DRAWINGS

Fig. 1 is a perspective view of a bag in accordance with the invention.
Fig. 2 is a top view of the bag of Fig. 1 in the open position.
Fig. 3 is a sectional view taken on Lines 3-3 of Fig. 1.
Fig. 4 is a partial side view showing an alternate construction of a portion of the bag.
Fig. 5 is a sectional view taken on Line 5-5 of Fig. 4.
Fig. 6 is an enlarged perspective detail view showing a closing device from the bag of Fig. 1.
Fig. 7 is a partial perspective view showing both the closure member of Fig. 6 in use and a configuration of the bag for use with a catheter.
Fig. 8 is a side view of another embodiment of the bag of the present invention, also showing an alternate means of closure of the bag.
Fig. 9 is a sectional view taken on Line 9-9 of Fig 8, also showing in phantom an alternate position.
Figure 10 is a top view of the bag of Figure 8 in the open position.
Figure 11 is a fragmentary side view, partially in section, showing an alternate closure means of the invention.
Figure 12 is a sectional view taken on Line 12-12 of Figure 11.
Figure 13 is a side view of another embodiment of the bag of the present invention, particularly adapted to be strapped to the user's leg.

### DESCRIPTION OF THE PREFERRED EMBODIMENTS

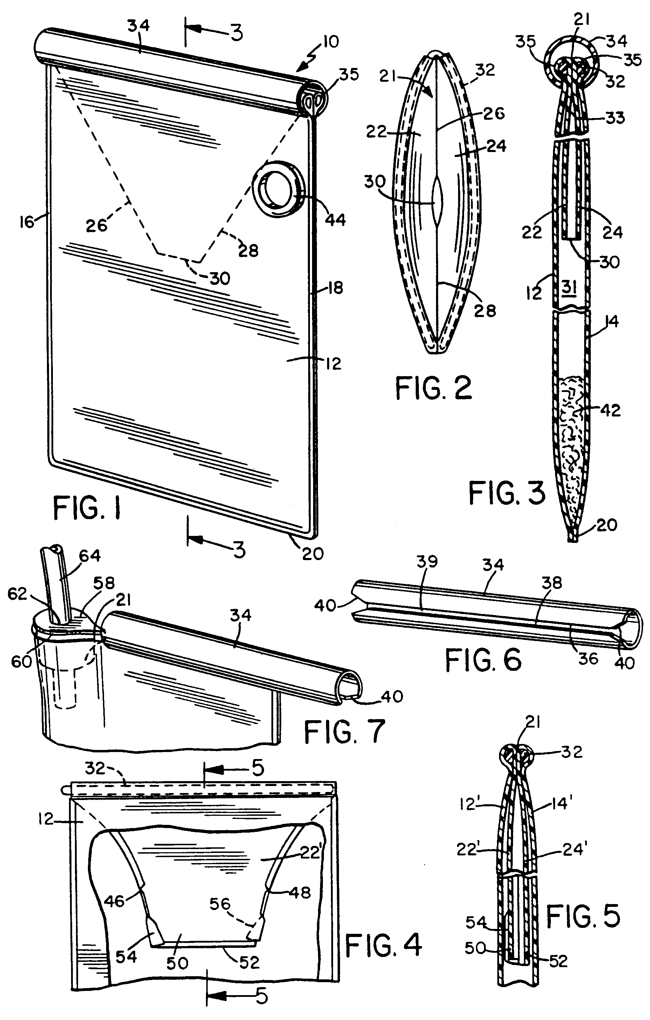

The invention herein will be best understood by reference to the drawings, beginning with Figure 1.

The embodiment shown in Figure 1 is a typical embodiment of the bodily fluids disposal bag of this invention, designated, generally, by the numeral 10. This bag is preferably constructed of a lightweight flexible plastic material that is impermeable and sufficiently thick and tough to resist accidental puncture under normal handling. The bag in its preferred embodiment as illustrated is a generally rectangular bag formed of a sheet of plastic or a pair of sheets of plastic, such as polyethylene, vinyl, mylar, or the like. The two-sheet embodiment comprises opposing rectangular sheets 12 and 14 secured or bonded together around three peripheral edges such as by heat sealing or other form of adhesive, forming seams along side edges 16 and 18 and bottom edge 20, leaving a top opening 21 as shown in Figures 2 and 3. The bag may also be formed of a single sheet cut and folded such along one side and the bottom with the sides subsequently sealed to leave the top opening 21.

Disposed within the interior of the bag, as shown in Figures 1, 8, and 13, is a funnel comprising a pair of opposed sheets 22 and 24 connected together along seams 26 and 28 and extending downwardly from the top opening of the bag to an opening 30 that opens into the interior chamber 31 of the bag. Alternatively the funnel may be formed of a single sheet of appropriate shape folded and sealed to have a top opening 33 and a bottom opening 30. Whether a funnel is formed of separate sheets or a single sheet, the top opening 33 is coextensive with the top opening 21 of the bag.

If desired, the sheets 22 and 24 may be extensions of the side panels 12 and 14 so that the panels forming the sides and funnel are formed of a single sheet cut to the configuration of a pair of rectangular side panels 12 and 14 positioned side by side with generally triangular panels 22 and 24 extending from the top thereof. The panels are then folded alongside edged 16 and 18 and the panels 22 and 24 folded over the stiffener member 32 and the panels are heat sealed along the edges 18 and 20 below the stiffener and along the edges 26 and 28 of the panels 22 and 24.

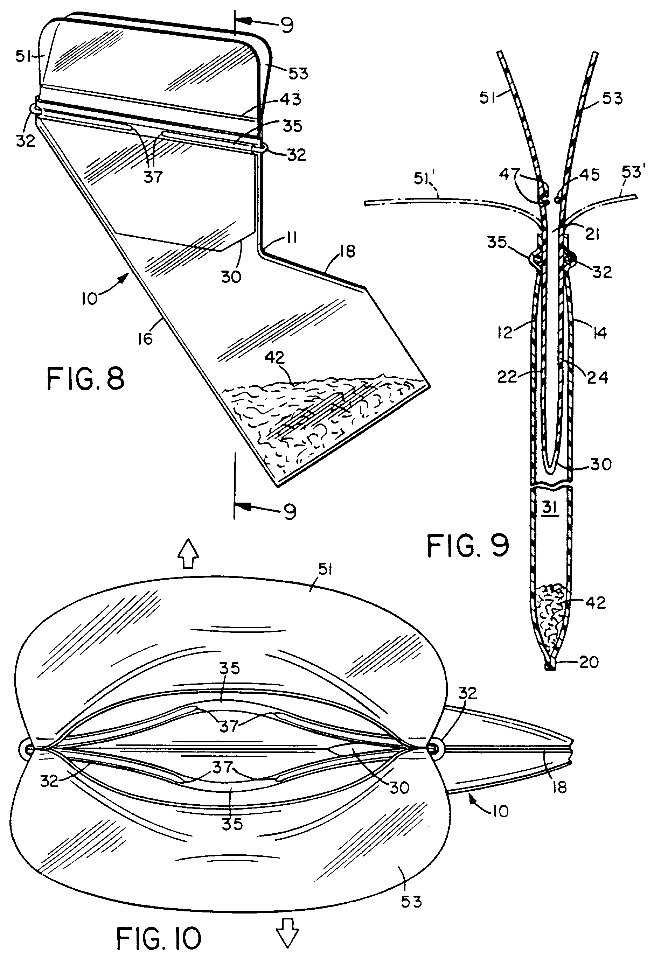

While the lower funnel opening 30 into the interior 31 of the bag 10 is shown in Figure 1 to be centrally located, it may be positioned at any point between sides 16 and 18. For instance, in Figure 8 the opening 30 is at one side of the bag. Normally for general use by both males and females the opening 30 will be at the center of the bag, but embodiments of the bag designed for specific situations or to be used exclusively by one or the other of the sexes may have the opening 30 offset accordingly.

Disposed around the periphery of opening 21 at the top of bag 10 is a stiffener 32 which is generally in the form of a bow or loop. A bow, as illustrated in Figure 2 is an elongated member made, for example, of a suitable piece of flexible plastic having sufficient memory such that when bent in the form of a bow as shown in Figure 2, will tend to bias the top edges of the panels forming the sides of the panels outward, thus acting as a toggle and forcing the bag open. The bowed stiffener 32 also aids in support the bag. The stiffener 32 is conveniently retained in place by having the top edges of the bag be folded over to form elongated cylindrical sleeves 35 into which the stiffener 32 can be placed. For many embodiments the stiffener 32 will be sealed in place by closure of the open ends of sleeves 35 as by heat sealing. Alternatively, however, as shown in Figure 8, the stiffener 32 may be in two separate pieces, each having a generally U shape. The two pieces are inserted respectively at opposite ends of the sleeves 35 and extend toward each other, either touching or being closely adjacent at their interior ends 37. This permits the stiffeners 32 to be removed if the user so chooses. It also permits a stiffener which may be broken to be replaced prior to use of the bag.

A convenient holding means in the form a metal eyelet or grommet 44 may be placed in an upper corner of the bag adjacent to the top to enable grasping and holding the bag while a clamp is secured thereto or while the bag is being used The grommet 44 is securely attached to the bag and forms a firm or rigid structure for grasping.

One means of closure of the bag comprises a clamp 34 which extends along the upper opening 21 of the bag just below the upper rim formed by sleeves 35. The clamp 34 is an elongated plastic or metal tube which is formed with a slot 39 extending longitudinally for the entire length of the clamp and being defined by opposed jaws 36 and 38 for engaging and biasing the two sides of the bag together in a sealed configuration. The ends of the slot 39 are notched as shown at 40 to provide ease of sliding the clamp 34 onto the top edge of the bag 10. The clamp 34 is placed on the bag 10 by engaging an edge 16 or 18 at notch 40 just below the sleeves 35 and stiffener 32 and sliding the clamp 34 across the top of the bag 10.

Wherever possible, and particularly along the top 21, it will be desirable to have the edge seams rounded to provide comfort in use and to prevent chafing the user's skin during contact with the bag.

An alternate form the bag 10 and means of closure is shown in figure 8. This configuration is in the general form of an L shape with a distinct notch 11 formed in the edge 18, with the other edge 16 disposed at an angle to form the L shape. This shape of bag would be conveniently used for instance by military pilots who are restricted in their movements within the close confined spaces of the aircraft cockpit. Also shown in the configuration of Figure 8 is an alternative form of closure of the bag which is in the form of a snap-and-seal zipper closure structure 43 formed by longitudinal rib 45 which interfits between two opposed longitudinal ribs 47 in a releasable fashion to form a seal. This type of seal, which does not require the use of clamp 34, is particularly useful in bags intended for use by persons in vehicles, particularly aircraft, for it integrates the sealing means directly into the bag structure and avoids the presence of a separate clamp. This is particularly desirable with military aircraft, where the pilot's attention should not be distracted by the need to keep track of a separate bag and clamp.

Also shown in Figures 8-10 is an alternate means of gripping the bag by the user which comprises adjacent extended flaps 51 and 53. These in turn are upwardly disposed extensions of sheets 22 and 24 which extend upwardly and outward of opening 21. These flaps 51 and 53 can be folded over and downward as shown in phantom in Figure 9 (as 51' and 53' respectively). The large flaps 51 and 53 provide a large surface which may be easily grasped by users in situations where it might not otherwise be convenient or easy to hold the bag. For instance, military pilot with their hands encased in flying gloves will find these extended surfaces more easily grasped than other alternate grasping means such as grommet 44. It is also anticipated that these large surfaces may be more convenient for use by children, women or those with physical infirmities such as arthritis of the hands.

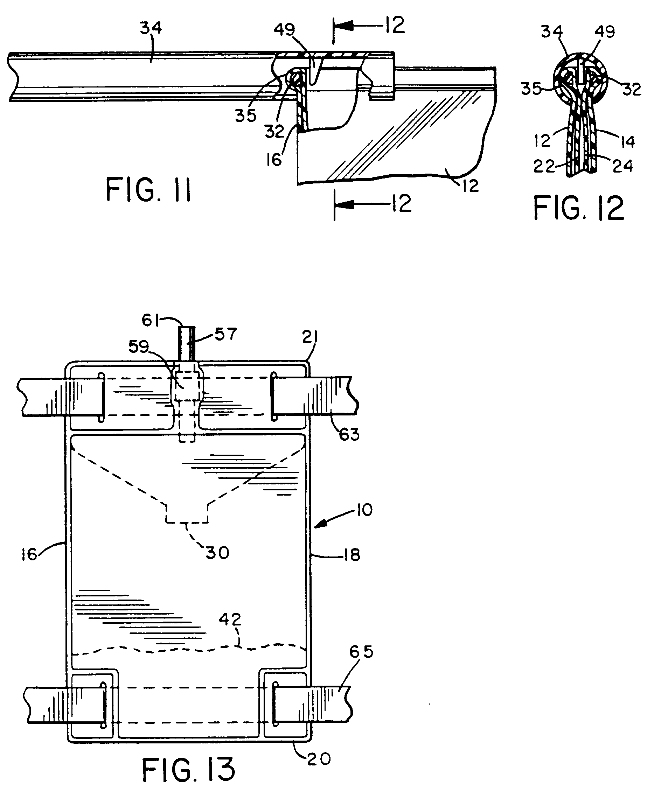

Another embodiment of clamp 34 is shown in Figures 11 and 12. This version of the clamp 34 includes an internal tang 49 which extends downwardly inside the hollow barrel of clamp 34 and engages the edge 16 or 18 and the outer curve of stiffener 32 when the clamp 34 is pulled to one side of the bag. This prevents the clamp from being pulled off of the bag and it also provides an effective handle in its extended position which the user may grasp during use of the bag.

A further embodiment of the bag 10 is shown in Figure 13 in which all sides 16, 18, and 20 are sealed and top 21 is sealed across most of its extent leaving only a small opening, preferably centrally located, to accommodate tube 57 (which normally contains one-way valve 59) and which at its upper end 61 is connected to or integrated with a catheter (not shown) which extends into a person's urinary tract. The bag 10 of Figure 13 can then be attached by means of straps 63 and 65 to the person's leg. Thus a person who is incontinent or because of surgery other medical procedures must be able to accommodate urinary drainage can still be ambulatory.

The bag preferably has a capacity to hold on the order of between ten to twenty fluid ounces (300-600 cc) of bodily fluids. This is more than adequate to accommodate the relief needs of one individual on a single occasion. The bags can of course be made larger if desired, but if made too large will be difficult for the user to handle. Smaller sizes of bags are not recommended, since they do not have sufficient volume to be adequate for a person's relief needs, except perhaps in the case of bags intended for use by infants or small children.

The bags 10 as noted above are normally made of an impervious flexible plastic material. Commonly, they will be of an opaque nature, either because of the material itself or by means of a colorant added to the plastic formulation. Alternatively, however, as shown in the configuration of Figure 9, the bag may be made of a clear plastic so that the contents can be viewed. This would be particularly advantageous in a medical situation where a physician or other medical attendant needs to be able to observe the patient's urine for signs of abnormalities. It is also of great significance for military pilots who would need to be able to detect blood in the urine signifying internal injuries after combat. In addition, it may be convenient to have the outer sides of the bag be of a soft or otherwise comfortable feeling material for comfort in those circumstances such as with use of the bag of Figure 13 where the user must keep the bag against his or her skin for a prolonged period.

Enclosed within the bag and critical to the function of the present invention is a quantity of an absorbent material 42 for absorbing the liquid contained within the bag. This absorbent material 42 contains a hydrophilic gellable material, usually a polymer, which is water activated and which gels very rapidly (normally within 30 seconds, and often much less than that) upon contact with a water-based liquid (such as urine or blood) and which by gelling completely absorbs and encapsulates all of the fluid. Such polymers are commercially available and are commonly found in a variety of known products, including disposable diapers and cleaning compositions. Typical examples include the acrylonitrile-based polymers described in Elias, Mega Molecules, pp. 157-158 (1987). The mixture 42 is preferably a complex mixture including not only the gellable material but also a material such as a protease enzyme to attack and break down the urine, blood or other bodily fluid to enhance the operation of the gellable material. Also included in the mixture 42 can be deodorants and fragrances. It is also possible to incorporate a biocide or antiviral material where it is expected that the bodily fluids may be contaminated as in the case of collection of blood from a surgery or medical procedure patient. The mixture 42 may be in any convenient physical form which can be placed into the bag 10; granular, powdered, foamed, matted, woven and fibrous forms are all suitable. We have successfully used a granular material commercially available under the trade name "Sanwet IM-5600" from Hoechst Celanese, Superabsorbent Material Division, of Portsmouth, Virginia. which is described as containing a starch grafted sodium polyacrylate. This product is a proprietary product and the exact identification of the components and formula is not available to applicants. The product has shown the property in our tests of gelling and sequestering all bodily fluids placed into test bags within no more than twenty seconds.

In the case of blood collection, it may be desirable to incorporate within the mixture 42 one or more testing materials which upon contact with the blood will show visible color changes indicative of specific blood disorders or the presence in the blood of specific organism. Similarly, in the case of urine collection, it is also possible to incorporate within the mixture 42 materials which with a similar color change will also indicate urinary tract infections, internal disorders which manifest signs in the urine or the presence of organisms such as bacteria or viruses in the urine. It is also contemplated to incorporate known pregnancy detectors of the type commonly used in home pregnancy test kits such that the bag when used by a female for urination, will provide an indication of whether or not the female is pregnant. Naturally , in all of these cases the bag material will be transparent so that the desired indicators can be readily observed. Also, of course, the various indicators must be compatible with the gellable absorption material and any enzyme present, so that the functions of all of the components will not be impaired by the presence of any other component.

Referring now to Figure 4, there is illustrated a modification of the front structure of the bag 10 to accommodate it for use as an air sickness or other motion sickness bag into which the susceptible person can vomit. Such a modification could replace the existing bags currently carried on airlines and provide for easier disposal. The construction of the bag of Figure 4 is basically the same as that of Figure 1 with the exception that the walls of the funnel 22' and 24' are connected along seams which stop at points 46 and 48, but the sides extend downwardly forming flaps 50 and 52. These flaps each have a stiffener at edges 54 and 56 respectively extending from the edge of the seam and forcing the excess material of one flap to curl over the other flap, thus forcing the flaps to close together. This forms a self sealing one-way valve such that back breathing or inhalation of the material 42 or the expelled vomit by the person is prevented.

Figure 7 illustrates means for adapting the bag to hospital use with a catheter. This system includes a plug 58 which has a generally teardrop shaped cross section and has a curved surface 60 for engaging and sealing to the curved portion of the opening 21 of the bag 10. The clamp 34 clamps against the plug 58 and holds it in the sealed position. The plug includes a central bore 62 through which is placed a catheter tube 64.

The bag is shown in the drawings as having a generally rectangular shape, whether straight or in the L shaped configuration. It will be evident that it may also be tapered, have a rounded or pointed bottom edge or have some other desired configuration, as long as the containment and disposal functions are adequately maintained.

The advantage of the present invention is over existing types of urine and other waste collection and disposal bags is evident. The presence of the rapidly gelling material allows for the completed collection and sequestration of the bodily fluids within a matter of seconds such that the bag of collected bodily fluids almost immediately becomes completely free of the possibility of leakage or spillage of any of the bodily fluids. Thus, the bag can be immediately closed as with the clamp 34 or the zipper closure 43 and be set aside for subsequent safe and convenient disposal. In this condition the bag is virtually impervious to accidental spillage of the contents. Thus, for instance, a pilot or airplane passenger can use the bag, seal it and within seconds set it aside with no concern that subsequent aerial maneuvers will cause any of the collected bodily fluids to be spilled within the interior of the aircraft. Similarly, a field worker can use the bag and again immediately seal it and set it aside with no concern that subsequent jostling or dropping of the bag will cause any spillage of the contents.

It will be evident that there are numerous embodiments of the present invention. Consequently, the above description is considered to be exemplary only and the full scope of the invention is to be determined solely by the appended claims.

## Claims

1. A containment bag for human bodily fluids, said containment bag comprising:
a bag having a hollow interior defined by two sides (12, 14) meeting at opposite edges (16, 18), a bottom (20) and a top (21) with said edges (16, 18) and bottom (20) sealed and said top (21) being at least partially open, and
funnel means (22, 24) having an open top (33), being secured to the bag (10) at the top of the bag and extending downwardly within the interior of the bag to a narrower open bottom (30) whereby fluid entering the open top (33) of the funnel means (22, 24) may be conducted into bag whilst the escape of fluid from within the bag through the funnel means (22, 24) is resisted,
the funnel means (22, 24) being arranged completely inside the interior of the bag (10);
the funnel means (22, 24) comprising flexible material which is attached at its top (33) to the top (21) of the bag (10); characterized in that:
gellable hydrophillic polymer material (42) is contained within the bag (10), said material becoming fully gelled within thirty seconds of contact when the fluid is deposited in the bag (10), said gelation serving to essentially completely sequester the fluid and to prevent the fluid from thereafter being expelled from the bag (10);
and closure means for closing the top of said bag after introduction of said bodily fluids into said bag.

2. A containment bag as in claim 1, wherein said hydrophilic material (42) is part of a mixture of materials which also contains at least one material selected from the group consisting of enzymes, deodorants, fragrances, human body abnormality indicators and pregnancy indicators.

3. A containment bag as in Claim 1 wherein said hydrophilic material (42) is in a powdered, matted, granular, fibrous, foamed, or woven physical form.

4. A containment bag as in Claim 3 wherein said hydrophilic material (42) is a powdered or granular form and has at least about 80% with particle sizes in the range of -40+120 mesh U.S. Sieve Series.

5. A containment bag as in Claim 4 wherein said hydrophilic material (42) also has at least about 80%-90% with particle sizes in the range of -40+20 mesh U.S. Sieve Series.

6. A containment bag as in Claim 5 wherein said hydrophilic material (42) also has at least about 50% with particle sizes in the range of -40+80 mesh U. S . Sieve Series.

7. A containment bag as in Claim 1 further comprising gripping means (44;51,53) attached to said bag (10) for gripping of said bag by the user thereof during use.

8. A containment bag as in Claim 7 wherein said gripping means comprises a grommet (44) disposed in the sides (12,14) of said bag (10).

9. A containment bag as in Claim 7 wherein said gripping means comprises flaps (51,53) extending outwardly from said top of said bag (10).

10. A containment bag as in Claim 1 wherein said closure means comprises an elongated closure member (34) which fits across said top of said bag (10) and contains opposed jaws (36,38) which contact the sides (12,14) of said bag adjacent said top and bias said sides into contact to close the top opening.

11. A containment bag as in Claim 10 wherein said elongated closure member (34) is moveable to one side of said top of said bag (10) to serve as a handle by which the user of said bag can grip the bag (10) during use.

12. A containment bag as in Claim 11 wherein said elongated closure member (34) contains a tang (49) on the inside thereof and projecting into the interior of said bag (10), said tang (49) when said member (34) is moved to one side of said top contacting the inside edge of the top opening of said bag (10) and preventing said member (34) from becoming disengaged from said bag (10).

13. A containment bag as in Claim 1 wherein said closure means comprises cooperating ribs (45,47) on the inside surfaces of the sides (12,14) or said bag (10) adjacent, which ribs interfit to close the top opening of said bag.

14. A containment bag as in Claim 1 wherein said bag further comprises means (63,65) for attachment to the body or limb of a user and conduit means comprising a catheter adapted for enabling said user to sue said bag (10) for urine collection while said user is ambulatory.

15. A containment bag as in Claim 1 wherein said funnel means (22,24) has its bottom opening (30) in the form of a self sealing one-way valve.

16. A containment bag as in Claim 1 wherein said bag (10) has the form of an L shape.

## Patentansprüche

1. Einschließungsbeutel für Körperflüssigkeiten des Menschen, wobei der Einschließungsbeutel umfaßt:
einen Beutel mit einem durch zwei Seiten (12, 14), welche an sich gegenüberliegenden Rändern (16, 18) zusammenstoßen, definierten Innenhohlraum, einem Boden (20) und einem Oberteil (21), wobei die Ränder (16, 18) und der Boden (20) verschlossen sind und das Oberteil (21) zumindest teilweise offen ist, und
Trichtermittel (22, 24) mit einem offenen Oberteil (33), welche an den Beutel (10) am Oberteil des Beutels befestigt sind und sich innerhalb des Innenraums des Beutels nach unten zu einem engeren offenen Boden (30) erstrecken, wodurch Flüssigkeit, welche in das offene Oberteil (33) der Trichtermittel (22, 24) eintritt, in den Beutel geführt werden kann, während einem Entweichen der Flüssigkeit von innerhalb des Beutels durch die Trichtermittel (22, 24) Widerstand entgegen gesetzt wird,
wobei die Trichtermittel (22, 24) vollständig innerhalb des Innenraums des Beutels (10) angeordnet sind;
die Trichtermittel (22, 24) flexibles Material umfassend, das an dem Oberteil (33) mit dem Oberteil (21) des Beutels (10) verbunden ist; dadurch gekennzeichnet, daß
gelierbares, hydrophiles Polymermaterial (42) innerhalb des Beutels (10) enthalten ist, wobei dieses Material innerhalb eines Kontaktes von 30 Sekunden vollständig geliert wird, wenn die Flüssigkeit in den Beutel (10) abgeschieden wird, wobei die Gelierung dazu dient, die Flüssigkeit im wesentlichen vollständig zu sequestrieren und die Flüssigkeit daran zu hindern, danach aus dem Beutel (10) ausgestoßen zu werden;
sowie Verschlußmittel zum Verschließen des Oberteils des Beutels nach Einführung der Körperflüssigkeiten in den Beutel.

2. Einschließungsbeutel nach Anspruch 1, wobei das hydrophile Material (42) Teil einer Mischung aus Materialien ist, welche ebenso mindestens ein Material enthält, gewählt aus der Enzyme, Desodoranzien, Duftstoffe, Abnormalitätsindikatoren für den menschlichen Körper und Schwangerschaftsindikatoren umfassenden Gruppe.

3. Einschließungsbeutel nach Anspruch 1, wobei das hydrophile Material (42) in einer pulverförmigen, mattierten bzw. verfilzten, granulären, faserförmigen, geschäumten oder gewobenen physikalischen Form vorliegt.

4. Einschließungsbeutel nach Anspruch 3, wobei das hydrophile Material (42) in einer pulverförmigen oder granulären Form vorliegt und mindestens etwa 80 % mit Teilchengrößen im Bereich von -40+120 mesh U.S. Sieve Series aufweist.

5. Einschließungsbeutel nach Anspruch 4, wobei das hydrophile Material (42) ebenso mindestens etwa 80 bis 90 % mit Teilchengrößen im Bereich von -40+20 mesh U.S. Sieve Series aufweist.

6. Einschließungsbeutel nach Anspruch 5, wobei das hydrophile Material (42) ebenso mindestens etwa 50 % mit Teilchengrößen im Bereich vom -40+80 mesh U.S. Sieve Series aufweist.

7. Einschließungsbeutel nach Anspruch 1, umfassend weiterhin Einspann- bzw. Klemmittel (44; 51, 53), welche an dem Beutel (10) befestigt sind, zum Einspannen bzw. Einklemmen des Beutels durch dessen Anwender während des Gebrauchs.

8. Einschließungsbeutel nach Anspruch 7, wobei die Einspann- bzw. Einklemmittel eine Durchführungshülse (44) umfassen, welche in den Seiten (12, 14) des Beutels (10) angeordnet ist.

9. Einschließungsbeutel nach Anspruch 7, wobei die Einspann- bzw. Einklemmittel Laschen (51, 53) umfassend, welche sich vom Oberteil des Beutels (10), nach außen erstrecken.

10. Einschließungsbeutel nach Anspruch 1, wobei die Verschlußmittel ein längliches Verschlußelement (34) umfassen, das entlang des Oberteils des Beutels (10) anliegt und gegenüberliegende Backen (36, 38) enthält, welche die Seiten (12, 14) des Beutels benachbart dem Oberteil kontaktieren und die Seiten zum Kontakt spannen, um die Öffnung des Oberteils zu verschließen.

11. Einschließungsbeutel nach Anspruch 10, wobei das längliche Verschlußelement (34) zu einer Seite des Oberteiles des Beutels (10) beweglich ist, um als Handgriff zu dienen, durch welchen der Anwender den Beutel (10) während des Gebrauchs greifen kann.

12. Einschließungsbeutel nach Anspruch 11, wobei das längliche Verschlußelement (34) einen Zapfen (49) aufdessen Innenseite enthält, der in das Innere des Beutels (10) ragt, wobei der Zapfen (10), wenn das Element (34) zu einer Seite des Oberteils bewegt wird, den Innenrand der Öffnung des Oberteils des Beutels (10) kontaktiert und verhindert, daß das Element (34) von dem Beutel (10) losgelöst wird.

13. Einschließungsbeutel nach Anspruch 1, wobei die Verschlußmittel zusammenwirkende Rippen (45, 47) auf den Innenflächen der benachbarten Seiten (12, 14) des Beutels (10) umfassen, welche Rippen ineinander passen, um die Öffnung des Oberteils des Beutels zu verschließen.

14. Einschließungsbeutel nach Anspruch 1, wobei der Beutel weiterhin Mittel (63, 65) zur Befestigung am Körper oder einem Glied eines Anwenders sowie Leitungsmittel, umfassend einen Katheter, der so angepaßt ist, daß es dem Anwender möglich ist, den Beutel (10) zur Urinsammlung zu tragen, während der Anwender ambulant ist, umfaßt.

15. Einschließungsbeutel nach Anspruch 1, wobei die Trichtermittel (22, 24) ihre Bodenöffnung (30) in Form eines selbstverschließenden Einwegventils aufweisen.

16. Einschließungsbeutel nach Anspruch 1, wobei der Beutel (10) eine L-Form aufweist.

## Revendications

1. Sac pour liquides corporels humains, le dit sac comprenant :
un sac présentant une partie intérieure creuse déterminée par deux côtés (12, 14) se rejoignant par leurs bords opposés (16, 18), un fond (20) et une partie supérieure (21) , les dits bords (16, 18) et le fond (20) étant scellés et la dite partie supérieure (21) étant au moins partiellement ouverte,
un dispositif formant entonnoir (22, 24), à partie supérieure ouverte (33), étant fixé au sac (10) à la partie supérieure de celui-ci et s'étendant vers le bas à l'intérieur du sac jusqu'à une partie inférieure étroite ouverte (30), de sorte que du liquide entrant par la partie supérieure ouverte (33) du dispositif formant entonnoir (22, 24) peut être conduit à l'intérieur du sac tandis que l'échappement de liquide depuis l'intérieur du sac à travers le dispositif (22, 24) formant entonnoir, se trouve empêché,
le dispositif formant entonnoir (22, 24) étant disposé complètement à l'intérieur du sac (10) ;
le dispositif formant entonnoir (22, 24) comprenant un matériau souple dont la partie supérieure (33) est fixée à la partie supérieure du sac (10) ; caractérisé en ce que :
un matériau polymère hydrophile gélifiable (42) est contenu à l'intérieur du sac (10), ce dit matériau se gélifiant totalement dans un temps de contact de trente secondes lorsque du liquide est introduit dans le sac (10), cette gélification servant essentiellement à confisquer totalement le liquide et à empêcher celui-ci d'être ensuite refoulé du sac (10) ;
et un dispositif de fermeture pour fermer la partie supérieure du dit sac après introduction des dits liquides corporels dans le sac.

2. Sac selon la revendication 1, dans lequel le dit matériau hydrophile (42) fait partie d'un mélange de matériaux contenant au moins un matériau choisi dans le groupe comprenant les enzymes, les désodorisants, les parfums, les indicateurs d'anomalies du corps humain et les indicateurs de grossesse.

3. Sac selon la revendication 1, dans lequel le dit matériau hydrophile (42) se présente sous une forme physique pulvérulente, nattée, granuleuse, en mousse ou tissée.

4. Sac selon la revendication 3, dans lequel le dit matériau hydrophile (42) est sous forme pulvérulente ou granuleuse, environ 80% au moins des dimensions de particule étant dans la plage de maille -40 à +120 échelle de crible U.S.

5. Sac selon la revendication 4, dans lequel le dit matériau hydrophile (42) présente aussi au moins 80% - 90% des dimensions de particule dans la plage de maille de -40 à +120 échelle de crible U.S.

6. Sac selon la revendication 5, dans lequel le dit matériau hydrophile (42) présente aussi au moins environ 50% des dimensions de particule dans la plage de maille de -40 à +80 échelle de crible U.S.

7. Sac selon la revendication 1, comprenant de plus un dispositif de prise (44 ; 51, 53) fixé au dit sac (10) pour la prise du sac par son utilisateur en cours d'utilisation.

8. Sac selon la revendication 7, dans lequel le dit dispositif de prise comprend une virole (44) disposée dans les côtés (12, 14) du dit sac (10).

9. Sac selon la revendication 7, dans lequel le dit dispositif de prise comprend des volets (51, 53) s'étendant vers l'extérieur depuis la dite partie supérieure du sac (10).

10. Sac selon la revendication 1, dans lequel le dit dispositif de fermeture comprend un élément de fermeture allongé (34) qui s'ajuste en travers de la dite partie supérieure du dit sac (10) et contient des mâchoires opposées (36, 38) qui engagent les côtés (12, 14) du dit sac au voisinage de la dite partie supérieure et contraignent les dits côtés à venir en contact pour fermer l'ouverture supérieure.

11. Sac selon la revendication 10, dans lequel le dit élément de fermeture allongé (34) est mobile vers un côté de la dite partie supérieure du sac (10) pour servir de poignée grâce à laquelle l'utilisateur peut saisir le sac (10) pendant l'utilisation.

12. Sac selon la revendication 11, dans lequel le dit élément de fermeture allongé (34) contient intérieurement un talon (49) qui fait saillie à l'intérieur du dit sac (10), le dit talon (49), lorsque le dit élément (34) se déplace vers un côté de la dite partie supérieure, engageant le bord intérieur de l'ouverture supérieure du dit sac (10) et empêchant le dit élément (34) de se dégager du dit sac (10).

13. Sac selon la revendication 1, dans lequel le dit dispositif de fermeture comprend, sur les surfaces intérieures des côtés (12, 14) du dit sac (10), des nervures destinées à coopérer ensemble, ces nervures s' ajustant l'une dans l'autre pour fermer l'ouverture supérieure du sac.

14. Sac selon la revendication 1, dans lequel le sac comprend de plus un dispositif (63, 65) de fixation au corps ou à un membre d'un utilisateur, et un dispositif de conduit comprenant un cathéter conçu pour permettre à l'utilisateur de solliciter le dit sac (10) pour une récolte d'urine pendant que le dit utilisateur est sur pieds.

15. Sac selon la revendication 1, dans lequel l'ouverture inférieure (30) du dit dispositif formant entonnoir (22, 24) présente la forme d'un clapet à une voie à auto-fermeture.

16. Sac selon la revendication 1, dans lequel le dit sac (10) a une forme en L.
